# EUROPEAN PATENT SPECIFICATION

(11) **EP 3 821 827 B1**
(45) Date of publication and mention of the grant of the patent: **15.10.2025**
(21) Application number: 19845304.5
(22) Date of filing: 30.07.2019
(51) Int. Cl.: A61B 17/135, A61B 90/00, A61B 17/00, A61B 17/132

(54) **HEMOSTASIS APPARATUS**
HÄMOSTASEGERÄT
APPAREIL D'HÉMOSTASE

(30) Priority: 01.08.2018 JP 2018145365
(43) Date of publication of application: 19.05.2021
(73) Proprietor: TERUMO Kabushiki Kaisha, Tokyo 151-0072 (JP)
(72) Inventor: HIOKI, Yuichi, Fujinomiya-shi Shizuoka 418-0015 (JP); OKAMURA, Ryo, Fujinomiya-shi Shizuoka 418-0015 (JP); WATANABE, Fumi, Fujinomiya-shi Shizuoka 418-0015 (JP); WADA, Satoshi, Fujinomiya-shi Shizuoka 418-0015 (JP); MIYASHITA, Masako, Fujinomiya-shi Shizuoka 418-0015 (JP)
(74) Representative: Casalonga
(86) International application number: PCT/JP2019/029819
(87) International publication number: WO 2020/027122

(56) References cited:
- EP-A1- 3 345 555
- WO-A1-2018/181314
- CN-A- 107 970 054
- JP-A- 2008 119 517
- US-A1- 2017 042 552
- US-A1- 2017 143 346
- US-A1- 2019 133 602

## Description

### Technical Field

The present invention relates to a hemostatic device.

### Background Art

As a catheter procedure, a procedure is known in which a blood vessel (for example, radial artery) in an arm of a patient is punctured, and various medical elongated bodies are introduced into the blood vessel via a puncture site formed in the blood vessel in the arm of the patient so as to perform treatment or therapy on a lesion area (see PTL 1 below). The catheter procedure utilizing the radial artery is referred to as transradial artery approach and is considered as a useful technique for, for example, coronary artery access and lower limb artery access.

A radial artery located in an arm of a human body is connected to a palmar artery which bypasses a hand side. Therefore, currently, as a new method of transradial artery approach, a catheter procedure using distal transradial approach (dTRA) has been attempted to access the palmar artery (including a distal radial artery) from an anatomical snuffbox located on a dorsal side of the hand or from a position around the snuffbox, and perform treatment through the vascular access site.
CN 107 970 054 A refers to a femoral artery puncture point aeration pressing type tourniquet in order to achieve a good hemostatic effect, convenient and quick operation, and at the same time facilitate the observation of the puncture site. JP 2008 119517 A relates to a hemostatic device that can easily be aligned for compressing a puncture site comprising a transparent balloon and a marker for aligning the central portion of the balloon with the site to be hemostatic.
US 2017/042552 A1 discloses methods and devices for obtaining patent hemostasis of the radial artery by compressing the uninstrumented ulnar artery to increase radial artery flow. US 2019/133602 A1 refers to a hemostasis device that may comprise a plurality of bands used to position and maintain a compression member over the puncture site.
WO 2018/181314 A1 relates to a tourniquet and hemostatic method that restricts the axial movement of the fixing part by a restriction part that is disposed between adjacent fingers of the hand.
US 2017/143346 A1 discloses methods and devices for obtaining patent hemostasis of the radial artery by compressing the uninstrumented ulnar artery to increase radial artery flow. EP 3 345 555 A1 provides a hemostatic device capable of enhancing hemostatic effect by suppressing a decrease in blood flow rate of a radial artery and reducing numbness or pain occurring due to pressing of an ulnar artery.

### Citation List

### Patent Literature

PTL 1: JP-A-2008-119517

### Summary of Invention

### Technical Problem

Blood vessels such as palmar arteries located in the hand are located in places where there are many movable parts such as fingers. For this reason, a shape around the puncture site in the hand changes with movement of the hand. Therefore, when hemostasis is performed on the puncture site, a pressing member placed on the hand is preferably an inflatable member that follows the movement of the hand and can easily adjust a compressive force on the puncture site.

However, when the pressing member is the inflatable member, the inflatable member exerts a force to inflate from the inside to the outside of the inflatable member in an inflated state. For this reason, in a hemostatic device having the inflatable member, when a shape around a hemostatic site changes due to the movement of the hand, and a large gap is created between the hemostatic device and the puncture site, the inflatable member rises, and it may not be possible to properly maintain the compressive force of the inflatable member on the puncture site. Therefore, when hemostasis is performed on the puncture site of the hand, the hemostatic device having the inflatable member needs to suppress rising by the inflatable member acting in a direction away from the puncture site and to appropriately secure the inflatable member to the puncture site. In this way, it is considered that the hemostatic device having the inflatable member can appropriately maintain the compressive force of the inflatable member on the puncture site even when the shape around the puncture site is changed by the movement of the hand.

In view of the above problems, an object of the invention is to provide a hemostatic device capable of suppressing rising of an inflatable member from a site where bleeding is to be stopped on a hand and appropriately maintaining a compressive force of the inflatable member on the site where bleeding is to be stopped on the hand even when a patient moves the hand while the inflatable member is inflated.

### Solution to Problem

A hemostatic device according to an aspect of the invention includes a covering member configured to be disposed to cover a site where bleeding is to be stopped on a hand of a patient, a securing member configured to secure the covering member in a state where the covering member covers the site where bleeding is to be stopped, and an inflatable member connected to the covering member and configured to be inflated by injection of a fluid, in which the covering member includes a main body to which the inflatable member is connected, a first arm portion protruding from the main body, and a second arm portion protruding from the main body in a direction different from a longitudinal direction of the first arm portion, the first arm portion is configured to be secured to the second arm portion by passing between fingers of the patient in a state where the second arm portion is wrapped around a limb of the patient, the second arm portion has a belt portion for wrapping around the limb of the patient and a support portion located on a straight line connecting the first arm portion and the inflatable member to connect the main body and the belt portion, and the belt portion is configured to be secured to the support portion in a state of being wrapped around the limb of the patient.

A hemostatic device according the invention includes a covering member configured to be disposed to cover a site where bleeding is to be stopped on a hand of a patient, a securing member configured to secure the covering member in a state where the covering member covers the site where bleeding is to be stopped, and an inflatable member connected to the covering member and configured to be inflated by injection of a fluid, in which the covering member includes a main body to which the inflatable member is connected, a first arm portion protruding from the main body, and a second arm portion protruding from the main body in a direction different from a longitudinal direction of the first arm portion, the first arm portion is configured to be secured to the second arm portion by passing between fingers of the patient in a state where the second arm portion is wrapped around a limb of the patient, the inflatable member has a first inflatable portion and a deformable auxiliary member having a smaller outer shape than an outer shape of the first inflatable portion, and the auxiliary member is disposed to overlap the first inflatable portion on a side of the first arm portion of the first inflatable portion.

### Advantageous Effects of Invention

The hemostatic device according to the aspect of the invention can secure the main body, to which the inflatable member is connected, to the site where bleeding is to be stopped by wrapping the belt portion of the second arm portion around the limb of the patient. In addition, in the hemostatic device, when the first arm portion is secured to the second arm portion by passing between the fingers of the patient, it is possible to prevent the inflatable member from rising on the distal side of the hand while preventing position shift of the inflatable member. Further, when the hemostatic device is attached to the hand of the patient, it is possible to prevent the inflatable member from rising on the proximal side of the hand by connecting the belt portion of the second arm portion to the support portion located on the straight line connecting the first arm portion and the inflatable member. For this reason, in the hemostatic device, even when the hand is moved in a state in which the inflatable member is inflated, by securing the first arm portion and the second arm portion and securing the belt portion and the support portion, rising of the distal side (fingertip side) and the proximal side (wrist side) of the main body to which the inflatable member is connected is prevented. Thus, it is possible to appropriately maintain the compressive force of the inflatable member on the site where bleeding is to be stopped on the hand.

The hemostatic device according to the invention can secure the main body, to which the first inflatable portion is connected, to the site where bleeding is to be stopped by wrapping the second arm portion around the limb of the patient. Further, the hemostatic device can prevent the inflatable portion from rising on the distal side of the hand while preventing the first inflatable portion from being shifted by securing the first arm portion to the second arm portion through between the fingers of the patient. Further, when the first inflatable portion inflates, the hemostatic device inhibits the first inflatable portion from rising in a direction away from the site where bleeding is to be stopped on the hand of the patient by the auxiliary member. For this reason, even when the hand is moved in a state in which the inflatable member is inflated, the hemostatic device can prevent rising on the distal side (fingertip side) of the main body to which the inflatable member is connected and the distal side (fingertip side) of the first inflatable portion at the distal side of the hand where the shape of the hand changes most when the finger is moved by securing the first arm portion and the second arm portion and pressing the first inflatable portion using the auxiliary member. Thus, it is possible to appropriately maintain the compressive force of the first inflatable portion on the site where bleeding is to be stopped on the hand. In addition, in the hemostatic device, the deformable auxiliary member is disposed on the distal side of the hand (first arm side of the first inflatable portion) where the shape of the hand changes most when the finger is moved. Thus, when the shape of the auxiliary member changes according to the change in the shape of the hand, it is possible to appropriately maintain the compressive force of the first inflatable portion on the puncture site of the hand while preventing the main body from rising.

### Brief Description of Drawings

[Fig. 1] Fig. 1 is a diagram illustrating a hemostatic device according to a first embodiment, and is a plan view seen from an outer surface side of a main body of a covering member.
[Fig. 2] Fig. 2 is an enlarged view illustrating a part of the covering member.
[Fig. 3] Fig. 3 is a cross-sectional view of the hemostatic device taken along arrow 3-3 illustrated in Fig. 2, and is a diagram illustrating an inflated state of an inflatable member.
[Fig. 4] Fig. 4 is a perspective view for description of a usage example of the hemostatic device according to the first embodiment.
[Fig. 5] Fig. 5 is a perspective view for description of a usage example of the hemostatic device according to the first embodiment.
[Fig. 6] Fig. 6 is a perspective view for description of a usage example of the hemostatic device according to the first embodiment.
[Fig. 7] Fig. 7 is a perspective view for description of a usage example of the hemostatic device according to the first embodiment.
[Fig. 8] Fig. 8 is a perspective view for description of a usage example of the hemostatic device according to the first embodiment.
[Fig. 9] Fig. 9 is a diagram schematically illustrating a part of a cross section taken along arrow 9-9 illustrated in Fig. 8.
[Fig. 10] Fig. 10 is a plan view of a hemostatic device according to an exemplary modification of the first embodiment not falling under the scope of the claims.
[Fig. 11] Fig. 11 is a plan view of a hemostatic device according to an exemplary embodiment not falling under the scope of the claims.
[Fig. 12] Fig. 12 is a plan view of a hemostatic device according to a first modification of the exemplary embodiment of Fig. 11 not falling under the scope of the claims.
[Fig. 13] Fig. 13 is a perspective view for description of a usage example of the hemostatic device according to the first modification of the exemplary embodiment of Fig. 12 not falling under the scope of the claims.
[Fig. 14] Fig. 14 is a perspective view for description of a usage example of the hemostatic device according to the first modification of the exemplary embodiment of Fig. 12 not falling under the scope of the claims.
[Fig. 15] Fig. 15 is a plan view of a hemostatic device according to a second modification of the exemplary embodiment of Fig. 11 not falling under the scope of the claims.
[Fig. 16] Fig. 16 is a perspective view for description of a usage example of the hemostatic device according to the second modification of the exemplary embodiment of Fig. 15 not falling under the scope of the claims
[Fig. 17] Fig. 17 is a perspective view for description of a usage example of the hemostatic device according to the second modification of the exemplary embodiment of Fig. 15 not falling under the scope of the claims.

### Description of Embodiments

Hereinafter, embodiments of the invention will be described with reference to the accompanying drawings. Note that the following description does not limit the technical scope or the meaning of terms described in the appended claims. In addition, the dimensions or scales on the drawings may be exaggerated for convenience of description, and different from actuality ones.

### <First embodiment>

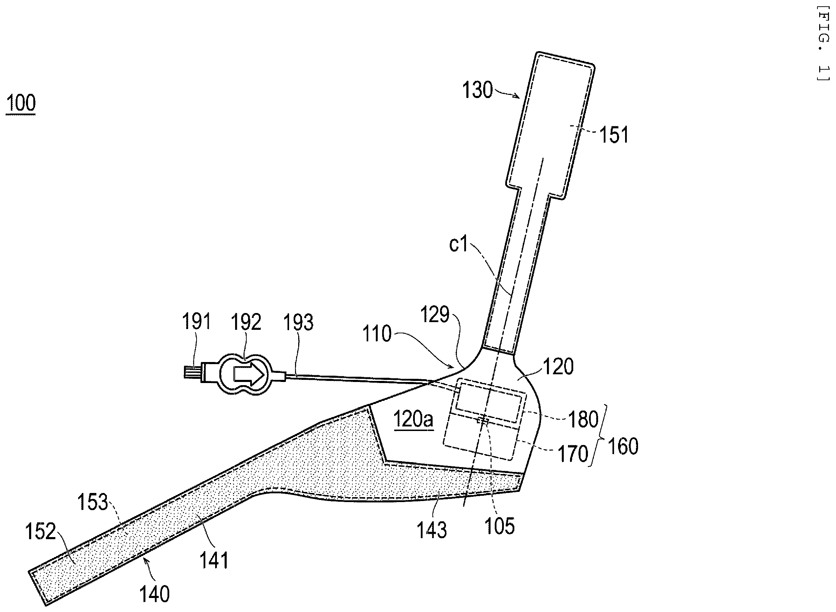

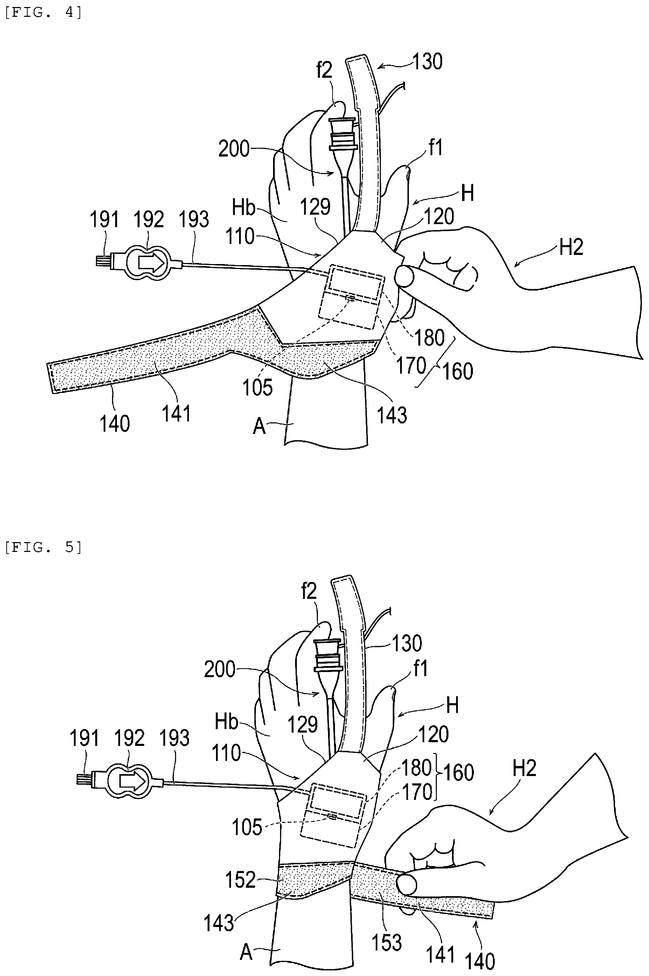

Figs. 1 to 3 are diagrams for description of a hemostatic device 100 according to a first embodiment, and Figs. 4 to 9 are diagrams for description of usage examples of the hemostatic device 100.

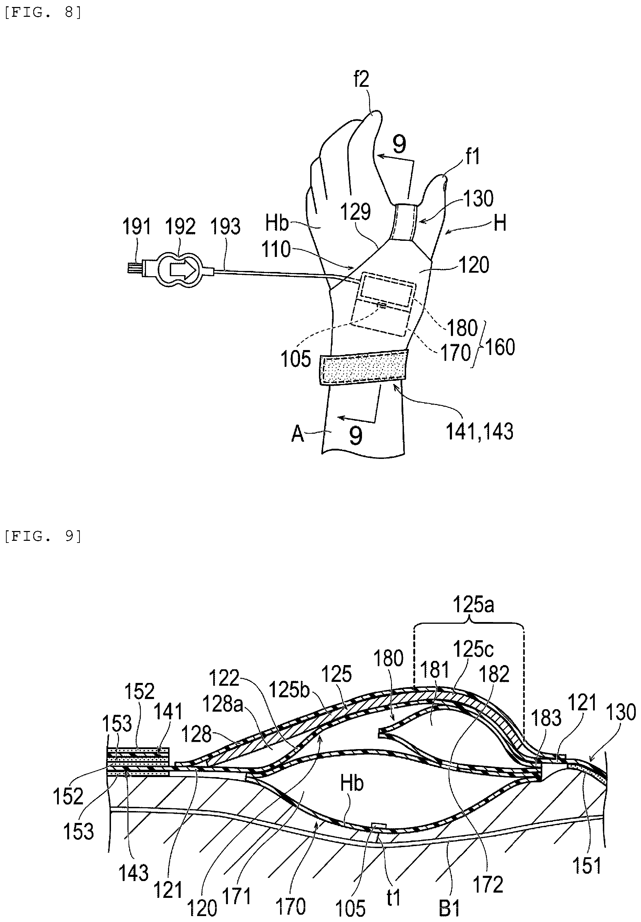

For example, as illustrated in Figs. 8 and 9, when removing a sheath tube of an introducer 200 indwelling at a puncture site t1 (corresponding to a site where bleeding is to be stopped) formed on a radial artery side (for example, an artery around an anatomical snuffbox or a distal radial artery running on a fingertip side of the snuffbox) of a palmar artery (deep palmar artery) B1 running on a side of a dorsal side Hb of a hand (for example, left hand or right hand) H located on the fingertip side of a forearm A of a patient, the hemostatic device 100 is used to perform hemostasis on the puncture site t1. Note that the anatomical snuffbox is a cavity in the hand H located on the radial side of the forearm A when the patient spreads a thumb f1 of the hand H.

In brief, as illustrated in Figs. 1, 2, and 9, the hemostatic device 100 includes a covering member 110 configured to be disposed to cover the puncture site t1 on the hand H of the patient, a plurality of securing members 151, 152, and 153 for securing the covering member 110 while the covering member 110 covers the puncture site t1, and an inflatable member 160 connected to the covering member 110 and configured to be inflated by injection of a fluid.

In the description of the present specification, a distal side of the hand H of the patient refers to a side where the fingertip of the hand H is disposed (upper side of Fig. 8).

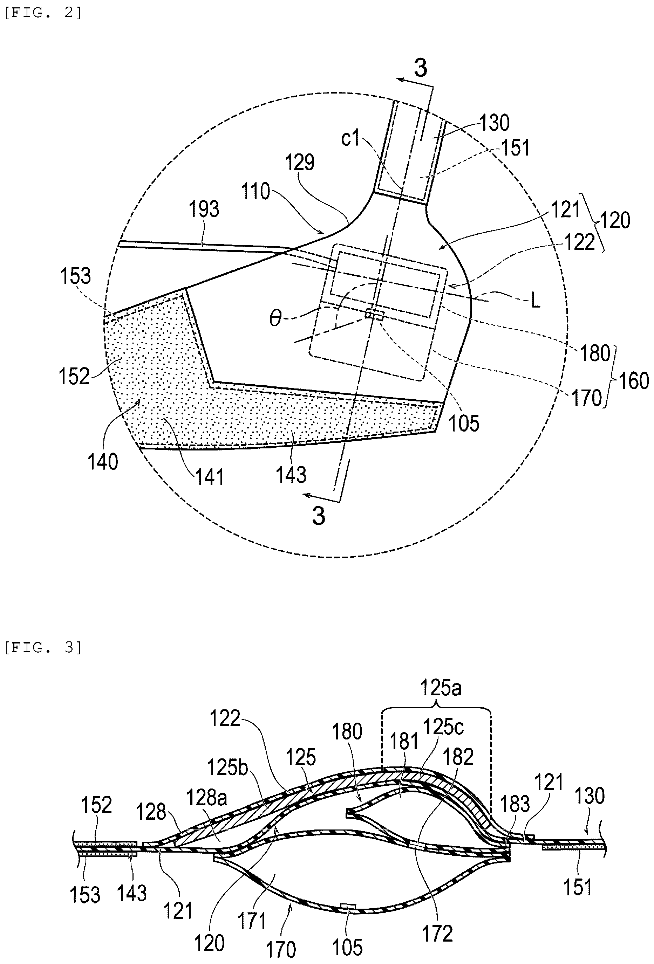

Figs. 1 and 2 are plan views of the hemostatic device 100 as viewed from an outer surface 120a side of a main body 120 of the covering member 110. Note that in each part of the hemostatic device 100, an "inner surface" is a surface on a side disposed to face a body surface of the patient when the hemostatic device 100 is attached to the patient, and an "outer surface" is a surface opposite to the inner surface (surface not disposed to face the body surface of the patient).

### (Covering member)

As illustrated in Fig. 1, the covering member 110 includes the main body 120 to which the inflatable member 160 is connected, a first arm portion 130 having a first securing member (corresponding to the securing member) 151 and protruding from the main body 120, and a second arm portion 140 having a second securing member (corresponding to the securing member) 152 and protruding from the main body 120 in a direction different from a longitudinal direction of the first arm portion 130.

As illustrated in Figs. 1, 2, and 8, when the hemostatic device 100 is attached to the hand H of the patient to perform hemostasis, the first arm portion 130 is secured to the second arm portion 140 by passing between fingers f1 and f2 of the patient in a state where the second arm portion 140 is wrapped around a limb of the patient.

As illustrated in Fig. 8, the limb around which the second arm portion 140 is wrapped is, for example, a portion including a part of the forearm A and a part of a wrist. In addition, a space between the fingers (inter-finger portion) through which the first arm portion 130 passes is, for example, between the thumb f1 and an index finger f2 of the hand H of the patient. However, a position of the limb around which the second arm portion 140 is wrapped or a position of the inter-finger portion through which the first arm portion 130 is passed is not particularly limited.

As illustrated in Fig. 8, the first arm portion 130 is formed of a flexible band-shaped member that can extend from the side of the dorsal side Hb of the hand H to a palm Hp side of the hand H of the patient by passing between the thumb f1 and the index finger f2 when the hemostatic device 100 is attached to the hand H of the patient. A wide portion for facilitating holding of the first arm portion 130 by fingers is provided at an end portion of the first arm portion 130 disposed on the opposite side from the main body 120.

The second arm portion 140 includes a belt portion 141 for wrapping the second arm portion 140 around the limb of the patient, and a support portion 143 located on a straight line c1, which connects the first arm portion 130 and the inflatable member 160, to connect the main body 120 of the covering member 110 and the belt portion 141 of the second arm portion 140.

As illustrated in Figs. 1 and 2, the straight line c1 can be defined as a straight line extending in parallel with an extending direction of the first arm portion 130 in a state where the first arm portion 130 and the second arm portion 140 are spread.

As illustrated in Fig. 8, the belt portion 141 of the second arm portion 140 is secured to the support portion 143 with the belt portion 141 wrapped around the limb of the patient. The belt portion 141 and the support portion 143 are secured by a third securing member 153 disposed on the belt portion 141 and the second securing member 152 disposed on the support portion 143 (see Fig. 9).

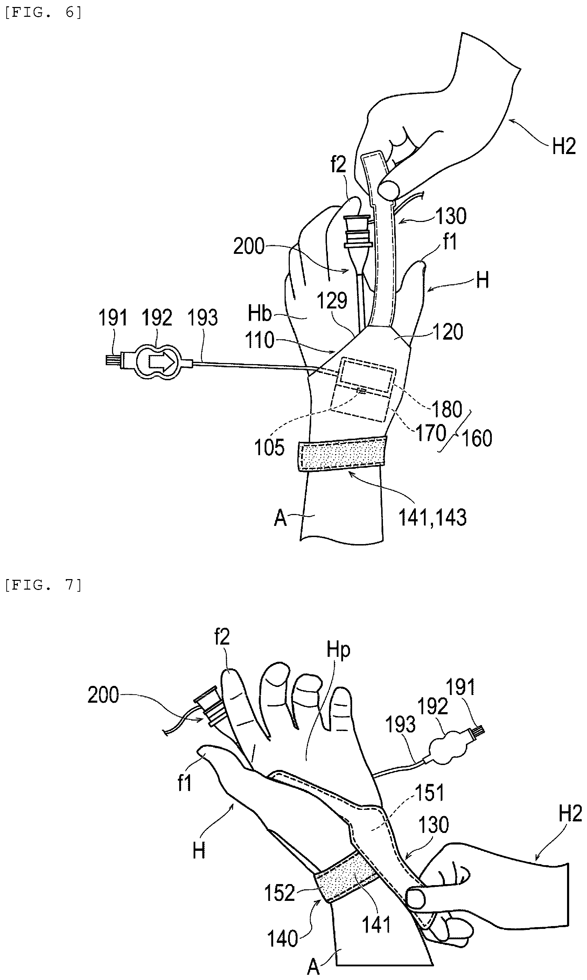

As illustrated in Figs. 5 and 7, the belt portion 141 is formed by a flexible band-shaped member that can be wrapped along a circumferential direction of the limb of the patient when the hemostatic device 100 is attached to the hand H of the patent. As illustrated in Fig. 5, when the hemostatic device 100 is attached to the hand H of the patent, the support portion 143 is disposed on a proximal side (wrist side when viewed from the hand H and lower side of Fig. 5) of the hand H of the patent with respect to the inflatable member 160. In the present embodiment, the belt portion 141 and the support portion 143 are integrally formed by one member. However, the belt portion 141 and the support portion 143 may be configured by connecting two or more members.

As illustrated in Fig. 1, the hemostatic device 100 can be configured such that an angle θ between the first arm portion 130 and the second arm portion 140 is 90° to 150° in a state where the arm portions 130 and 140 are extended substantially linearly before attaching the hemostatic device 100 to the patient. However, a direction in which each of the arm portions 130 and 140 protrudes from the main body 120 is not particularly limited as long as the inflatable member 160 can be disposed at the puncture site t1 in a state where the first arm portion 130 is disposed between predetermined fingers and the second arm portion 140 is wrapped around the limb.

For example, a proximal end of the first arm portion 130 (end portion of the first arm portion 130 on the main body 120 side) can be disposed at a position 200 mm to 250 mm away from a marker portion 105 of the main body 120. In addition, for example, a width of the first arm portion 130 can be formed to be 5 mm to 20 mm. In addition, a proximal end of the second arm portion 140 (end portion of the second arm portion 140 on the main body 120 side) can be disposed at a position 250 mm to 350 mm away from the marker portion 105 of the main body 120.

As illustrated in Figs. 2 and 3, the main body 120 of the covering member 110 has a first region 121 and a second region 122 which is formed in a part different from the first region 121 and on which the inflatable member 160 is disposed.

As illustrated in Fig. 3, the second region 122 of the main body 120 has a support member 125 having a higher rigidity than that of the first region 121 of the main body 120. Here, as illustrated in Fig. 2, the first region 121 of the main body 120 is disposed to surround the support member 125 having a higher rigidity than that of the first region 121. That is, the support member 125 is surrounded by the first region 121 that is more flexible than the support member 125. Therefore, when the inflatable member 160 (a first inflatable portion 170 and a second inflatable portion 180) located on the inner surface side of the support member 125 inflates in the second region 122, an inflating force from the inside to the outside of the inflatable member 160 is hardly transmitted from the support member 125 to the first region 121, and thus it is possible to suppress formation of a gap between a surface of the dorsal side Hb of the hand H of the patient and the main body 120 at a peripheral edge of the main body 120.

As illustrated in Fig. 2, the first region 121 of the main body 120 is disposed to surround the periphery of the second region 122 of the main body 120. As illustrated in Fig. 3, the support member 125 is inserted into an insertion portion 128a disposed on an outer surface side of the second region 122 (surface opposite to a side where each of the inflatable portions 170 and 180 is disposed in the second region 122, which is a surface on an upper side of Fig. 3).

The insertion portion 128a is a space formed between the cover member 128 disposed to cover a part of the outer surface of the main body 120 and the main body 120. Note that the insertion portion 128a is not illustrated except for the drawings of Figs. 3 and 9.

An opening (not illustrated) is formed between the main body 120 and the insertion portion 128a on a side portion side intersecting the straight line c1 on the plan view illustrated in Fig. 2. The insertion portion 128a communicates with the outside through the opening. The support member 125 can be inserted into the insertion portion 128a through the opening.

The cover member 128 can be connected to the covering member 110, for example, by adhesion or welding.

As illustrated in Fig. 3, the support member 125 has a curved portion 125a formed on the first arm portion 130 side. The curved portion 125a has a cross-sectional shape that protrudes upward so as to be separated from the main body 120 on the cross-sectional view illustrated in Fig. 3. The first arm portion 130 side means a side close to the first arm portion 130 with reference to a center position (position where the marker portion 105 is disposed in the present embodiment) on the plan view of the inflatable member 160 illustrated in Fig. 2.

The support member 125 has an inclined portion 125b extending from the curved portion 125a toward the support portion 143 side. The inclined portion 125b extends substantially linearly toward the support portion 143 side.

Note that, for example, the support member 125 may be disposed on the inner surface side of the main body 120 of the covering member 110 (surface on which each of the inflatable portions 170 and 180 is disposed, which is a surface on a lower side of Fig. 3). When the support member 125 is disposed in this way, each of the inflatable portions 170 and 180 can be secured to the support member 125, for example. Further, connection of the cover member 128 to the main body 120 can be omitted.

In addition, the peripheral edge of the main body 120 includes a sheath indwelling portion 129 having a shape recessed in a plan view toward the curved portion 125a side of the support member 125 at a position on the distal side (fingertip side) of an extension line of a straight line L1 (see Fig. 2) passing through an apex 125c (a portion where the curved portion 125a is most distant from an inner surface of the inflatable member 160 in a state before the inflatable member 160 is inflated) of the curved portion 125a of the support member 125 between the first arm portion 130 and the second arm portion 140. In addition, the curved portion 125a of the support member 125 forms a convex portion protruding in a direction away from the inflatable member 160 on the main body 120 of the covering member 110 in the state before the inflatable member 160 is inflated. For this reason, when the inflatable member 160 is inflated, the sheath indwelling portion 129 is recessed toward the curved portion 125a side of the support member 125 forming the convex portion on the main body 120, so that a distance between the peripheral edge of the main body 120 and the curved portion 125a is shortened. Thus, a gap portion is likely to be formed between the surface of the dorsal side Hb of the hand H of the patient and the covering member 110. Therefore, an operator attaches the hemostatic device 100 such that the sheath tube of the introducer 200 (see Figs. 5 and 6) is located at the sheath indwelling portion 129 of the main body 120, so that the sheath tube can be easily removed even after the inflatable member 160 of the hemostatic device 100 is inflated. Note that when the inflatable member 160 is inflated, the curved portion 125a side of the support member 125 wraps the end portion of the inflatable member 160 on the first arm portion 130 side, so that the compressive force of the inflatable member 160 is directed toward the center side of the support member 125. Thus, the compressive force of the inflatable member 160 is inhibited from escaping to the outside of the support member 125. For this reason, in the sheath indwelling portion 129, even when the inflatable member 160 inflates, and the gap portion is formed around the sheath indwelling portion 129 between the surface of the dorsal side Hb of the hand H of the patient and the covering member 110 so that the sheath tube can be easily removed, a decrease in the compressive force on the puncture site t1 by the inflatable member 160 is suppressed. Thus, it is possible to maintain the compressive force of the inflatable member 160 suitable for the puncture site t1.

The first region 121 and the second region 122 of the main body 120 are integrally formed by one member. However, the first region 121 and the second region 122 may be configured by connecting different members.

In the second region 122 of the main body 120, the cover member 128, the support member 125, the first inflatable portion 170, and the second inflatable portion 180, a portion overlapping the marker portion 105 in the plan view illustrated in Fig. 2 and surroundings thereof are preferably transparent (including colored transparent, colorless transparent, and translucent). By adopting such a configuration, the operator can visually recognize the puncture site t1 from the outer surface side of the main body 120 even when the marker portion 105 is superposed on the puncture site t1.

In the present embodiment, the covering member 110 includes the main body 120, the first arm portion 130, and the second arm portion 140 (the belt portion 141 and the support portion 143), each of which is a separate member. When respective portions of the covering member 110 are configured as separate members in this way, each of the main body 120, the first arm portion 130, and the second arm portion 140 can be connected by, for example, adhesion, welding, etc.

Note that the main body 120 is preferably made of a material different from that of the first arm portion 130 and the second arm portion 140. In this way, the main body 120 is easily deformed due to a change in physical property at each boundary between the first arm portion 130 and the second arm portion 140, and thus the first arm portion 130 and the second arm portion 140 can be easily disposed on the patient in a state where the main body 120 is disposed at the puncture site t1 formed on the hand H of the patient. Further, a material of the main body 120 is preferably more rigid than materials of the first arm portion 130 and the second arm portion 140. In this way, when the inflatable member 160 is inflated, the main body 120 can suppress rising of the main body 120 by the inflatable member 160 due to the rigidity of the main body 120. In addition, the first arm portion 130 and the second arm portion 140 are configured to be more flexible than the main body 120, so that when the hemostatic device 100 is attached to the patient, the covering member 110 can be easily attached along the shape of the hand H of the patient.

Further, in the present embodiment, in the covering member 110, the main body 120, the first arm portion 130, and the second arm portion 140 (the belt portion 141 and the support portion 143) are configured as different members, respectively. However, in the covering member 110, any part of the main body 120, the first arm portion 130, and the second arm portion 140 may be integrally formed of one member.

The material used for the main body 120 of the covering member 110 is not particularly limited. Examples thereof include polyvinyl chloride, polyolefin such as polyethylene, polypropylene, polybutadiene, or ethylene-vinyl acetate copolymer (EVA), polyester such as polyethylene terephthalate (PET) or polybutylene terephthalate (PBT), polyvinylidene chloride, silicone, polyurethane, various thermoplastic elastomers such polyamide elastomer, polyurethane elastomer, and polyester elastomer, nylon, nylon elastomer, or any combination thereof (blended resin, polymer alloy, laminate, etc.).

The material used for the cover member 128 is not particularly limited, and examples thereof include the same materials as those exemplified as the material of the covering member 110.

It is preferable that the material used for the support member 125 has a higher rigidity than that of the material used for the first region 121 of the main body 120 of the covering member 110. Examples of such a material include acrylic resin, polyvinyl chloride (especially hard polyvinyl chloride), polyolefin such as polyethylene, polypropylene, or polybutadiene, polystyrene, poly-(4-methylpentene-1), polycarbonate, ABS resin, polymethylmethacrylate (PMMA), polyacetal, polyacrylate, polyacrylonitrile, polyvinylidene fluoride, ionomer, acrylonitrile-butadiene-styrene copolymer, polyester such as polyethylene terephthalate (PET) or polybutylene terephthalate (PBT), butadiene-styrene copolymer, aromatic or aliphatic polyamide, fluorine-based resin such as polytetrafluoroethylene, etc.

The material used for the first arm portion 130 and the second arm portion 140 of the covering member 110 is not particularly limited. Examples thereof may include the same materials as the materials exemplified as the main body 120 of the covering member 110, woven fabric, nonwoven fabric, felt, cloth, knitted fabric, and paper.

### (Securing member)

As illustrated in Figs. 1, 2, and 3, the first securing member 151 is disposed on the inner surface of the first arm portion 130. The first securing member 151 is formed on a female side of a surface fastener.

The second securing member 152 is disposed on an outer surface of the second arm portion 140 (an outer surface of the belt portion 141 and an outer surface of the support portion 143). The second securing member 152 is formed on a male side of the surface fastener.

The third securing member 153 is disposed on an inner surface of the second arm portion 140 (an inner surface of the belt portion 141 and an inner surface of the support portion 143). The third securing member 153 is formed on the female side of the surface fastener.

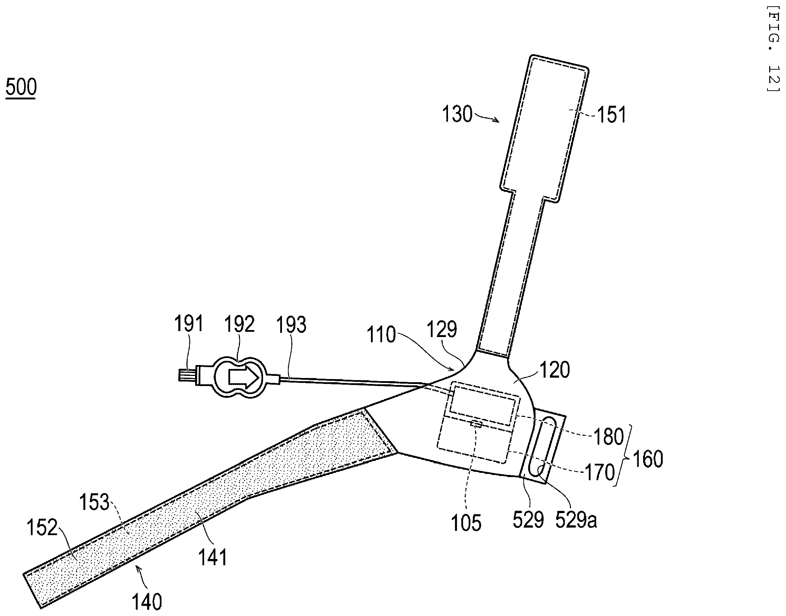

Note that a specific configuration of each of the securing members 151, 152, and 153 is not limited as long as the second arm portion 140 wrapped around the limb of the patient can be secured to the support portion 143, and the first arm portion 130 passed between the thumb f1 and the index finger f2 can be secured to the second arm portion 140. For example, when each of the securing members 151, 152, and 153 includes the surface fastener, the male side and the female side of the surface fastener may be interchanged. The surface fastener is a fastener that is removable in terms of surface, and is, for example, Magic Tape (registered trademark) or Velcro (registered trademark). In addition, for example, each of the securing members 151, 152, and 153 may be a snap, a button, a clip, a member 529 having a hole 529a as described in the embodiments described later (see Fig. 12), etc.

### (Inflatable member)

As illustrated in Figs. 2 and 3, the inflatable member 160 has the first inflatable portion 170 and a deformable auxiliary member 180 having a smaller outer shape (outer shape on the plan view of Fig. 2) than that of the first inflatable portion 170.

The auxiliary member 180 is disposed to overlap the first inflatable portion 170 on the first arm portion 130 side of the first inflatable portion 170. The first arm portion 130 side means a side close to the first arm portion 130 with reference to a center position (position where the marker portion 105 is disposed in the present embodiment) on the plan view of the inflatable member 160 illustrated in Fig. 2.

The first inflatable portion 170 has a lumen 171 into which a fluid can be injected, and a communication hole 172 formed at a position facing the auxiliary member 180.

The auxiliary member 180 is a second inflatable portion that is inflated by injection of a fluid. Hereinafter, the auxiliary member 180 is referred to as a second inflatable portion.

The second inflatable portion 180 has a lumen 181 into which a fluid can be injected, and a communication hole 182 formed at a position facing the first inflatable portion 170.

The lumen 181 of the second inflatable portion 180 communicates with the lumen 171 of the first inflatable portion 170 through the communication hole 182 of the second inflatable portion 180 and the communication hole 172 of the first inflatable portion 170.

The inflatable member 160 is fixed to the covering member 110 on the curved portion 125a side of the support member 125. As illustrated in Fig. 3, the second inflatable portion 180 is fixed to the inner surface of the covering member 110 (the inner surface of the main body 120 of the covering member 110) . An end portion 183 located on the first arm portion 130 side of the second inflatable portion 180 is fixed to the inner surface of the covering member 110. In addition, the first inflatable portion 170 is connected to the second inflatable portion 180 near a central portion excluding a peripheral edge of the second inflatable portion 180. In the first inflatable portion 170, only a certain range around each communication hole 182 located near the central portion of the second inflatable portion 180 is fixed to the second inflatable portion 180. For this reason, the first inflatable portion 170 is not directly connected to the covering member 110, and is indirectly connected to the covering member 110 via the second inflatable portion 180.

Note that the inflatable member 160 may be fixed to the inner surface of the covering member 110 at a position different from that in Fig. 3 as long as the inflatable member 160 is fixed to the covering member 110 on the curved portion 125a side of the support member 125. Specifically, a part or the whole of the outer surface side of the second inflatable portion 180 may be fixed to the inner surface of the covering member 110 on the curved portion 125a side of the support member 125. Even in such a configuration, in the hemostatic device 100, the compressive force on the first arm portion 130 side of the inflatable member 160 becomes stronger due to securing of the support member 125 and the covering member 110. Thus, even when a gap is generated between the first arm portion 130 and the surface of the hand H due to movement of a finger of the patient or bending of the wrist, it is possible to prevent a decrease in compressive force of the inflatable member 160.

The first inflatable portion 170 has a substantially square shape on the plan view illustrated in Fig. 2. The second inflatable portion 180 has a substantially rectangular shape that includes a set of long sides having substantially the same length as that of one side of the first inflatable portion 170 and a set of short sides having a length which is approximately half a length of one side of the first inflatable portion 170 .

The lumen 181 of the second inflatable portion 180 communicates with a lumen of a tube 193 for supplying a fluid such as air to the second inflatable portion 180. As illustrated in Fig. 2, the tube 193 is connected to the second inflatable portion 180 on the second arm portion 140 side of the second inflatable portion 180. The tube 193 is pulled out to the outside of the main body 120 through the inner surface side of the main body 120 of the covering member 110. A position where the tube 193 is pulled out from the second inflatable portion 180 is not particularly limited. However, as illustrated in Fig. 2, when the tube 193 is pulled out to the second arm portion 140 side, so that the hemostatic device 100 is attached to the patient, the tube 193 is disposed laterally to the hand H (in a direction intersecting a direction in which the finger of the hand H extends) (see Fig. 5). For this reason, when the hemostatic device 100 is attached to the patient, the tube 193 can be prevented from interfering with the introducer 200.

As illustrated in Figs. 2 and 3, the hemostatic device 100 has the marker portion 105 for aligning the inflatable member 160 with respect to the puncture site t1.

The marker portion 105 is disposed at a position corresponding to a substantially center position (center position on the plan view illustrated in Fig. 2) of the first inflatable portion 170.

As illustrated in Fig. 3, for example, the marker portion 105 can be disposed on an inner surface of a side surface (inner surface) of the first inflatable portion 170 disposed to face the body surface. However, for example, the marker portion 105 may be disposed on an internal surface of a surface (outer surface) opposite to the side surface of the first inflatable portion 170 disposed to face the body surface or an external surface thereof, an internal surface or an external surface of the main body 120 of the covering member 110, an internal surface or an external surface of the support member 125, an internal surface or an external surface of the cover member 128, etc. In addition, when a center portion of the first inflatable portion 170 and an end portion of the second inflatable portion 180 (end portion on the support portion 143 side) are disposed to overlap each other on the cross-sectional view illustrated in Fig. 3, the marker portion 105 may be disposed on an external surface of the end portion of the second inflatable portion 180.

For example, the marker portion 105 preferably includes a transparent central portion and a colored linear frame portion surrounding the central portion. In this way, the operator can dispose the marker portion 105 at the puncture site t1 while confirming the puncture site t1 through the transparent central portion of the marker portion 105. For this reason, the operator can easily dispose the center position of the first inflatable portion 170 at the puncture site t1 using the marker portion 105. Note that, for example, the marker portion 105 may be formed only by the colored central portion without having the frame portion. Further, a specific shape and color of the marker portion 105, a formation method on each portion of the hemostatic device 100, etc. are not particularly limited.

In the present embodiment, the first inflatable portion 170 is formed of two sheet-shaped members. For example, the first inflatable portion 170 can be formed by forming the lumen 171 between two sheet-shaped members formed in a substantially rectangular shape and bonding outer peripheral edges of the two sheet-shaped members in this state. Similarly to the first inflatable portion 170, the second inflatable portion 180 can be formed of two substantially rectangular sheet-shaped members bonded together.

A method of bonding the sheet-shaped members forming the first inflatable portion 170, and a method of bonding the sheet-shaped members forming the second inflatable portion 180 are not particularly limited. For example, it is possible to adopt adhesion or welding. Further, a method of connecting the second inflatable portion 180 and the main body 120 of the covering member 110 is not particularly limited. For example, adhesion or welding can be adopted. Further, a method of securing the periphery of the communication hole 172 of the first inflatable portion 170 and the periphery of the communication hole 182 of the second inflatable portion 180 is not particularly limited. For example, adhesion or welding can be adopted.

Note that the first inflatable portion 170 and the second inflatable portion 180 may not have a structure in which a plurality of sheet-shaped members is bonded. The first inflatable portion 170 and the second inflatable portion 180 may be formed of, for example, one bag-shaped member in which a space into which a fluid can flow is formed.

A material used for the first inflatable portion 170 and the second inflatable portion 180 is not particularly limited, and examples thereof may include the same materials as those exemplified as the material of the covering member 110.

### (Injection portion)

As illustrated in Fig. 1, the hemostatic device 100 has an injection portion 191 for injecting a fluid into the inflatable member 160 (the first inflatable portion 170 and the second inflatable portion 180).

The injection portion 191 includes a connector having an incorporated check valve (not illustrated) . A syringe (not illustrated) can be connected to the injection portion 191.

A cushioning member 192 having an inflatable space is disposed between the injection portion 191 and the inflatable member 160. The cushioning member 192 includes a flexible bag-shaped member having a space formed inside. Note that as illustrated in Fig. 1, the cushioning member 192 may be provided with an arrow-shaped marker indicating a direction in which the syringe is inserted into the injection portion 191.

The injection portion 191 is connected to one end side of the cushioning member 192. A lumen of the injection portion 191 communicates with a space in the cushioning member 192. However, while the check valve incorporated in the injection portion 191 is closed, communication between the lumen of the injection portion 191 and the space in the cushioning member 192 is cut off.

A flexible tube 193 is connected to the other end side of the cushioning member 192. A lumen of the tube 193 communicates with the space in the cushioning member 192. Further, in the tube 193, the other end portion opposite to one end portion connected to the cushioning member 192 is connected to the second inflatable portion 180. The lumen of the tube 193 communicates with the lumen 181 of the second inflatable portion 180.

For example, the other end portion of the tube 193 can be connected to the second inflatable portion 180 using an adhesive, etc. while being interposed between the two sheet-shaped members forming the second inflatable portion 180. Note that, in the sheet-shaped members forming the second inflatable portion 180, for example, convex portions partially protruding outward from the sheet-shaped members may be formed at parts interposing the tube 193 therebetween.

To inflate the first inflatable portion 170 and the second inflatable portion 180, the operator inserts a distal tubular portion of a syringe (not illustrated) into the injection portion 191 to open the check valve. The operator injects air in the syringe into the lumen 181 of the second inflatable portion 180 by pushing a plunger of the syringe with the check valve of the injection portion 191 open.

When air is injected into the lumen 181 of the second inflatable portion 180, the second inflatable portion 180 inflates. Further, the air injected into the lumen 181 of the second inflatable portion 180 flows into the lumen 171 of the first inflatable portion 170 via the communication hole 182 of the second inflatable portion 180 and the communication hole 172 of the first inflatable portion 170. When air flows into the lumen 171 of the first inflatable portion 170, the first inflatable portion 170 inflates. When the first inflatable portion 170 and the second inflatable portion 180 inflate, the cushioning member 192 communicating with the lumen 181 of the second inflatable portion 180 via the tube 193 inflates.

The space in cushioning member 192 and the lumen 181 of the second inflatable portion 180 are in communication with each other via the tube 193 at all times. The injection portion 191 maintains the check valve incorporated in the injection portion 191 in a closed state when the syringe is not inserted into the injection portion 191 to prevent air from leaking from the injection portion 191. For this reason, when the internal pressure of the inflatable member 160 increases due to movement of the hand H of the patient, etc., the air in the lumen 171 of the first inflatable portion 170 and the lumen 181 of the second inflatable portion 180 moves to the cushioning member 192 side which is not pressed against the puncture site t1 by the covering member 110. In this way, the compressive force applied to the puncture site t1 by the inflatable member 160 is adjusted, and thus the compressive force to the puncture site t1 by the inflatable member 160 can be appropriately maintained.

When the operator contracts the first inflatable portion 170 and the second inflatable portion 180, the operator inserts the distal tubular portion of the syringe into the injection portion 191 and pulls the plunger of the syringe. By performing the above operation, the operator can discharge the air in the first inflatable portion 170 and the air in the second inflatable portion 180 to the syringe.

Note that when the operator, etc. inflates the inflatable member 160, the operator, etc. can visually confirm that the first inflatable portion 170 and the second inflatable portion 180 can be pressurized without leakage of air by confirming expansion of the cushioning member 192.

Next, a usage example of the hemostatic device 100 will be described with reference to Figs. 4 to 9.

Fig. 4 illustrates a state after performing various procedures by inserting the sheath tube of the introducer 200 into the distal radial artery side of the palmar artery B1 via the puncture site t1 (see Fig. 9) formed on the dorsal side Hb of the hand (left hand) H of the patient. In addition, Fig. 4 illustrates a state in which a part of the sheath tube of the introducer 200 is pulled out from the puncture site t1 after completing the above procedure.

At the start of hemostasis, as illustrated in Fig. 4, the operator, etc. disposes the main body 120 of the covering member 110 so as to overlap the side of the dorsal side Hb of the hand H. In this instance, the marker portion 105 formed at a substantially center position of the first inflatable portion 170 is disposed at the puncture site t1. Note that a hand H2 illustrated in Figs. 4 to 7 indicates a hand of the operator, etc. who attaches the hemostatic device 100 to the patient.

Subsequently, as illustrated in Fig. 5, the operator, etc. wraps the belt portion 141 of the second arm portion 140 along the limb of the patient to overlap the belt portion 141 on the support portion 143. The operator, etc. brings the third securing member (female side of the surface fastener) 153 disposed on the inner surface side of the belt portion 141 into contact with the second securing member (male side of the surface fastener) disposed on the outer surface side of the support portion 143 to secure the support portion 143 and the belt portion 141 via the respective securing members 152 and 153.

Subsequently, as illustrated in Fig. 6, the operator, etc. passes the first arm portion 130 between the thumb f1 and the index finger f2 of the hand H of the patient. Then, as illustrated in Fig. 7, the operator, etc. overlaps a part of the first arm portion 130 on the belt portion 141 near the limb of the patient on the palm Hp side of the hand H of the patient. The operator, etc. brings the first securing member (female side of the surface fastener) 151 disposed on the inner surface side of the first arm portion 130 into contact with the second securing member (male side of the surface fastener) disposed on the outer surface side of the belt portion 141 to secure the belt portion 141 and the first arm portion 130 via the respective securing members 151 and 152.

Subsequently, the operator, etc. connects the syringe to the injection portion 191, and injects air into the second inflatable portion 180 to inflate the first inflatable portion 170 and the second inflatable portion 180. In the hemostatic device 100, when the first inflatable portion 170 and the second inflatable portion 180 are inflated, the first inflatable portion 170 applies a compressive force to the puncture site t1. After inflating the respective inflatable portions 170 and 180, as illustrated in Figs. 8 and 9, the operator, etc. removes the sheath tube of the introducer 200 from the puncture site t1.

The operator, etc. confirms that there is no bleeding from the puncture site t1 while hemostasis is performed using the hemostatic device 100. when there is bleeding from the puncture site t1, the amount of air injected into the respective inflatable portions 170 and 180 is adjusted.

After a certain period of time passes after start of hemostasis, the operator, etc. gradually depressurizes the respective inflatable portions 170 and 180 to confirm that hemostasis at the puncture site t1 is properly performed. After the hemostasis at the puncture site t1 is completed, the operator, etc. sufficiently depressurizes the respective inflatable portions 170 and 180. Then, the operator, etc. releases securing of the hemostatic device 100 by the first arm portion 130 and the second arm portion 140, and removes the hemostatic device 100 from the hand H of the patient.

As illustrated in Fig. 8, during hemostasis, the hemostatic device 100 is firmly secured to the hand H of the patient by the first arm portion 130 passed between the thumb f1 and index finger f2 of the hand H of the patient and the second arm portion 140 wrapped around the limb of the patient. For this reason, since each finger is not covered by the covering member 110 in a state where the hemostatic device 100 is attached to the hand H, the patient can freely move the finger while hemostasis is performed.

The operator, etc. can easily attach the hemostatic device 100 to the hand H of the patient by proceeding with an attachment operation according to the procedure described with reference to Figs. 4 to 7. Therefore, the operator can easily attach the hemostatic device 100 to the patient in a short time using the first arm portion 130 and the second arm portion 140. Further, the hemostatic device 100 can be secured to the hand H of the patient by securing the arm portions 130 and 140 to each other using the securing members 151, 152 and 153 disposed on the respective arm portions 130 and 140. Therefore, the hemostatic device 100 can prevent damage to the skin of the patient during attachment when compared to a hemostatic device secured to the hand H or the forearm A of the patient using a seal member provided with an adhesive material.

Further, since the hemostatic device 100 includes the first inflatable portion 170 as a member that applies a compressive force to the puncture site t1, the compressive force can be easily adjusted by adjusting the internal pressure of the first inflatable portion 170. Further, in the hemostatic device 100, even when the first inflatable portion 170 is deformed so as to change the internal pressure following movement of the hand H when the patient moves the hand H, the deformable second inflatable portion 180 mitigates the change in the internal pressure of the first inflatable portion 170. Therefore, the first inflatable portion 170 has a high following property to movement of the hand H of the patient, and compression on the puncture site t1 by the first inflatable portion 170 can be appropriately maintained.

Further, the hemostatic device 100 is configured to cover only a part of the dorsal side Hb of the hand H of the patient by the covering member 110, and is not configured to cover the entire hand H. For this reason, when the patient moves the hand H in a state where the hemostatic device 100 is attached to the hand H of the patient, it is possible to prevent movement of the hand H of the patient from being transmitted to the entire hemostatic device 100. Therefore, the hemostatic device 100 can suppress position shift from the hand H of the patient when the patient moves the hand H in the state where the hemostatic device 100 is attached to the hand H of the patient.

Note that, for example, the hemostatic device 100 can be attached to the right hand of the patient without changing the configuration of the hemostatic device 100. When the hemostatic device 100 is attached to the right hand of the patient, an attachment procedure is substantially the same as the procedure illustrated in Figs. 4 to 7.

Hereinafter, effects of the present embodiment will be described.

The hemostatic device 100 according to the present embodiment includes the covering member 110 configured to be disposed to cover the puncture site t1 on the hand H of the patient, the securing members 151, 152, and 153 for securing the covering member 110 in a state where the covering member 110 covers the puncture site t1, and the inflatable member 160 connected to the covering member 110 and configured to be inflated by injection of a fluid. The covering member 110 includes the main body 120 to which the inflatable member 160 is connected, the first arm portion 130 protruding from the main body 120, and the second arm portion 140 protruding from the main body 120 in a direction different from the longitudinal direction of the first arm portion 130. The first arm portion 130 is configured to be secured to the second arm portion 140 by passing between the fingers f1 and f2 of the patient in a state where the second arm portion 140 is wrapped around the limb of the patient, the second arm portion 140 includes the belt portion 141 for wrapping around the limb of the patient and the support portion 143 located on the straight line c1 connecting the first arm portion 130 and the inflatable member 160 to connect the main body 120 and the belt portion 141, and the belt portion 141 is configured to be secured to the support portion 143 in a state of being wrapped around the limb of the patient.

The hemostatic device 100 configured as described above can secure the main body 120, to which the inflatable member 160 is connected, to the puncture site t1 by wrapping the belt portion 141 of the second arm portion 140 around the limb of the patient. Further, in the hemostatic device 100, when the first arm portion 130 is secured to the second arm portion 140 by passing between the fingers f1 and f2 of the patient, it is possible to prevent the inflatable member 160 from rising on the distal side of the hand H while preventing position shift of the inflatable member 160. Further, when the hemostatic device 100 is attached to the hand H of the patient, it is possible to prevent the inflatable member 160 from rising on the proximal side of the hand H by connecting the belt portion 141 of the second arm portion 140 to the support portion 143 located on the straight line c1 connecting the first arm portion 130 and the inflatable member 160. For this reason, in the hemostatic device 100, even when the patient moves the hand H in a state in which the inflatable member 160 is inflated, by securing the first arm portion 130 and the second arm portion 140 and securing the belt portion 141 and the support portion 143, rising of the distal side (fingertip side) and the proximal side (wrist side) of the main body 120 to which the inflatable member 160 is connected is prevented. Thus, it is possible to appropriately maintain the compressive force of the inflatable member 160 on the puncture site t1 formed on the dorsal side Hb of the hand H of the patient.

Further, the main body 120 of the covering member 110 has the first region 121 and the second region 122 which is formed in a part different from the first region 121 and in which the inflatable member 160 is disposed. The second region 122 has the support member 125 having a higher rigidity than that of the first region 121. Therefore, the hemostatic device 100 can prevent the inflatable member 160 from rising from the dorsal side Hb of the hand H of the patient by the support member 125 while hemostasis is performed. As a result, the hemostatic device 100 can suitably apply a compressive force from the inflatable member 160 to the puncture site t1 while being attached to the hand H of the patient.

Further, the first region 121 of the main body 120 is disposed to surround the periphery of the second region 122 of the main body 120. The support member 125 is inserted into the insertion portion 128a disposed on the outer surface side of the second region 122. Since the support member 125 is surrounded by the first region 121 which is more flexible than the support member 125, when the inflatable member 160 located in the second region 122 is inflated, a force expanding from the inside to the outside of the inflatable member 160 is hardly transmitted from the support member 125 to the first region 121. Therefore, the hemostatic device 100 can suppress formation of a gap between the surface of the dorsal side Hb of the hand H and the main body 120 at the peripheral edge of the main body 120. Further, by disposing the support member 125 to cover the outer surface side of the second region 122 of the main body 120, it is possible to effectively prevent the inflatable member 160 from rising from the dorsal side Hb of the hand H of the patient. Further, the hemostatic device 100 can be equipped with the support member 125 by a simple operation of inserting the support member 125 into the insertion portion 128a.

Further, the support member 125 has the curved portion 125a formed on the first arm portion 130 side of the support member 125. For this reason, in the support member 125, when the inflatable member 160 is inflated, a direction in which the inflatable member 160 applies a compressive force to the hand H of the patient is directed in an oblique direction toward the puncture site t1. For this reason, the hemostatic device 100 can more effectively apply a compressive force to the puncture site t1. Further, when the curved portion 125a of the support member 125 wraps the end portion of the inflatable member 160 on the first arm portion 130 side, the inflatable member 160 directs the compressive force toward the center side of the support member 125, and thus the compressive force of the inflatable member 160 is inhibited from escaping to the outside of the support member 125. As a result, even in a case where a gap is generated between the first arm portion 130 and the hand H of the patient, when the curved portion 125a presses the inflatable member 160 against the skin of the hand H of the patient, it is possible to prevent the inflatable member 160 from rising, and it is possible to suppress a decrease in the compressive force of the inflatable member 160 applied to the puncture site t1.

Further, the inflatable member 160 is secured to the covering member 110 on the curved portion 125a side of the support member 125. For this reason, in the inflatable member 160 (the first inflatable portion 170 and the second inflatable portion 180), by securing the inflatable member 160 and the support member 125, position shift of the inflatable member 160 with respect to the curved portion 125a of the support member 125 is prevented, and thus the compressive force of the curved portion 125a on a portion close to the first arm portion 130 becomes stronger. As a result, in the hemostatic device 100, even when a gap is generated between the first arm portion 130 and the surface of the hand H due to bending of the thumb f1 or the wrist of the patient, it is possible to prevent a decrease in the compressive force by the inflatable member 160.

Further, the inflatable member 160 has the first inflatable portion 170 and the deformable auxiliary member 180 having a smaller outer shape than that of the first inflatable portion 170, and the auxiliary member 180 is disposed to overlap the first inflatable portion 170 on the first arm portion 130 side of the first inflatable portion 170. When the first inflatable portion 170 inflates, the second inflatable portion 180 inhibits the first inflatable portion 170 from rising from the body surface of the patient. Therefore, when the first inflatable portion 170 inflates, the first inflatable portion 170 can effectively apply the compressive force to the puncture site t1. Further, when the patient bends a finger (for example, the thumb f1) or the wrist downward (to the palm Hp side of the hand H), the hemostatic device 100 maintains a state in which the auxiliary member 180 is deformed by following movement of the finger or the wrist and the auxiliary member 180 applies the compressive force to the puncture site t1. Further, in the hemostatic device 100, when the patient bends a finger (for example, the thumb f1) or the wrist upward (to the side of the dorsal side Hb of the hand H), the auxiliary member 180 is deformed following movement of the finger or the wrist, and the compressive force applied from the auxiliary member 180 to the puncture site t1 is weakened. Therefore, while hemostasis is performed by the hemostatic device 100, the hemostatic device 100 can maintain a state in which an appropriate compressive force is applied to the puncture site t1 while allowing the patient to move the finger or the wrist relatively freely.

Further, the auxiliary member 180 is the second inflatable portion 180 configured to be inflated by injection of a fluid, and the lumen 181 of the second inflatable portion 180 communicates with the lumen 171 of the first inflatable portion 170. Since the auxiliary member 180 includes the second inflatable portion 180 which is inflatable, it is possible to improve the following property of the second inflatable portion 180 to movement of the hand H. Further, since the lumen 171 of the first inflatable portion 170 and the lumen 181 of the second inflatable portion 180 communicate with each other, the first inflatable portion 170 and the second inflatable portion 180 can be easily inflated.

Further, the hemostatic device 100 has the injection portion 191 for injecting a fluid into the inflatable member 160. The cushioning member 192 having an inflatable space is disposed between the injection portion 191 and the inflatable member 160. In the hemostatic device 100, when the patient moves the hand H while the hemostatic device 100 is attached to the patient, the inflatable member 160 (the first inflatable portion 170 and the second inflatable portion 180) compressing the puncture site t1 on the hand H is deformed. When there is no escape place for air in the lumen of the inflatable member 160 (the lumen 171 of the first inflatable portion 170 and the lumen 181 of the second inflatable portion 180) during deformation of the inflatable member 160, deformation of the inflatable member 160 is hindered. Therefore, the patient has a limited movable range of the hand H. The cushioning member 192 included in the hemostatic device 100 allows air to move from the lumen of the inflatable member 160 to the cushioning member 192 when the patient moves the hand H. Therefore, it is possible to prevent the movable range from being restricted by the inflatable member 160 when the patient moves the hand H. Note that when the patient returns the hand H from the deformed state to the original state, air moves from the cushioning member 192 to the inflatable member 160. Therefore, the compressive force can be effectively applied to the puncture site t1 from the inflatable member 160.

### (Modification)

Next, a description will be given of a modification of the hemostatic device according to the first embodiment described above, but not falling under the scope of the claims. In description of the modification, detailed description of the configuration, etc. previously described in the first embodiment will be omitted. In addition, content not particularly described in the description of the modification can be regarded as the same as that in the first embodiment.

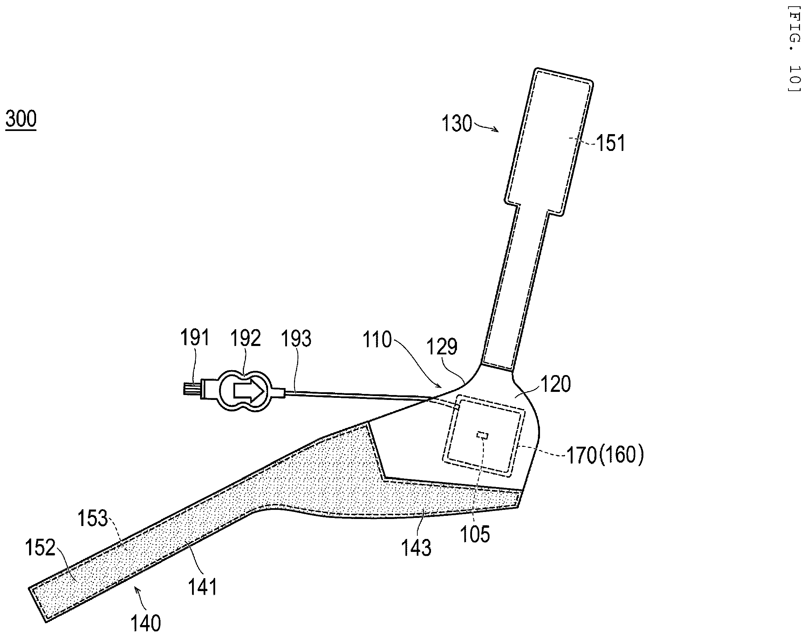

Fig. 10 illustrates a hemostatic device 300 according to the modification. In the hemostatic device 300 according to the modification, an inflatable member 160 only includes a first inflatable portion 170. That is, the inflatable member 160 does not include the second inflatable portion 180. Similarly to the hemostatic device 100 described above, by securing the hemostatic device 300 configured in this way to the hand H of the patient using respective arm portions 130 and 140 and a support portion 143, it is possible to prevent the inflatable member 160 from rising from the hand H during hemostasis. As a result, in the hemostatic device 300, the inflatable member 160 can effectively apply a compressive force to the puncture site t1.

Note that in the hemostatic device 300 according to the modification, the first inflatable portion 170 is directly fixed to an inner surface of a covering member 110. Further, a tube 193 is connected so as to communicate with a lumen 171 of the first inflatable portion 170. Other configurations of the hemostatic device 300 are substantially the same as those of the hemostatic device 100 described above.

### (Exemplary embodiment)

Next, a description will be given of a hemostatic device according to an exemplary embodiment not falling under the scope of the claims. In description of the exemplary embodiment, detailed description of the configuration, etc. previously described in the first embodiment will be omitted. In addition, content not particularly described in the description of the exemplary embodiment can be regarded as the same as that in the first embodiment.

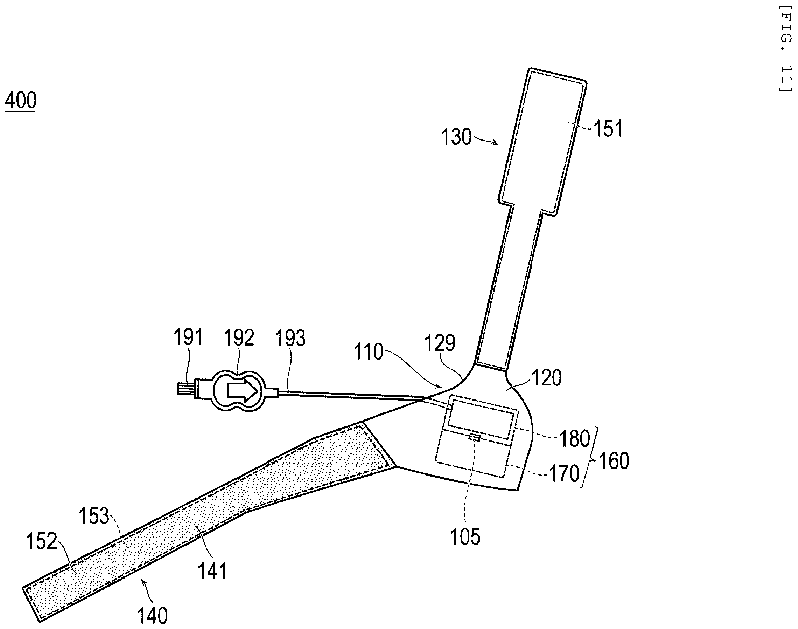

Fig. 11 illustrates a hemostatic device 400 according to the exemplary embodiment not falling under the scope of the claims. In the hemostatic device 400 according to the exemplary embodiment, a second arm portion 140 does not include the support portion 143. In this respect, the hemostatic device 400 differs from the hemostatic device 100 according to the first embodiment.

As the time of attaching the hemostatic device 400 to the patient, the operator wraps a belt portion 141 of the second arm portion 140 around the limb of the patient. In this instance, the operator brings a third securing portion (female side of a surface fastener) 153 disposed on an inner surface of the belt portion 141 into contact with a second securing portion (male side of the surface fastener) 152 disposed on an outer surface of the belt portion 141, thereby securing the respective securing members 152 and 153. The operator, etc. holds the belt portion 141 in a state of being wrapped around the limb of the patient by securing the respective securing members 152 and 153. Subsequently, the operator can attached the hemostatic device 400 to the hand H of the patient by securing the first arm portion 130 to the second arm portion 140 according to a similar procedure (see Figs. 6 and 7) to the procedure described in the above embodiment.

The hemostatic device 400 according to the exemplary embodiment includes a covering member 110 configured to be disposed to cover the puncture site t1 on the hand H of the patient, securing members 151, 152, and 153 for securing the covering member 110 in a state where the covering member 110 covers the puncture site t1, and an inflatable member 160 connected to the covering member 110 and configured to be inflated by injection of a fluid. The covering member 110 includes a main body 120 to which the inflatable member 160 is connected, a first arm portion 130 protruding from the main body 120, and a second arm portion 140 protruding from the main body 120 in a direction different from a longitudinal direction of the first arm portion 130. The first arm portion 130 is configured to be secured to the second arm portion 140 by passing between the fingers f1 and f2 of the patient in a state where the second arm portion 140 is wrapped around the limb of the patient. The inflatable member 160 has the first inflatable portion 170 and the deformable auxiliary member 180 having a smaller outer shape than that of the first inflatable portion 170, and the auxiliary member 180 is disposed to overlap the first inflatable portion 170 on the first arm portion 130 side of the first inflatable portion 170.

In the hemostatic device 400 according to the exemplary embodiment, the main body 120 to which the first inflatable portion 170 is connected can be secured to the puncture site t1 by wrapping the second arm portion 140 around the limb of the patient. Further, the hemostatic device 400 can prevent the first inflatable portion 170 from rising on the distal side of the hand H while preventing the first inflatable portion 170 from being shifted by securing the first arm portion 130 to the second arm portion 140 through between the fingers f1 and f2 of the patient. Further, when the first inflatable portion 170 inflates, the hemostatic device 400 inhibits the first inflatable portion 170 from rising in a direction away from the puncture site t1 on the hand H of the patient by the auxiliary member 180. As a result, the hemostatic device 400 can appropriately secure the first inflatable portion 170 and the auxiliary member 180 to the puncture site t1. Further, in the hemostatic device 400, when the first inflatable portion 170 inflates, the auxiliary member 180 inhibits the first inflatable portion 170 from rising in the direction away from the puncture site t1 of the hand H of the patient, and thus it is unnecessary to secure to firmly tighten the first arm portion 130 passing between the fingers f1 and f2 of the patient to the second arm portion 140. In this way, the patient can move the finger or the wrist relatively freely in a state where the hemostatic device 400 is attached.

Further, when the patient bends a finger (for example, the thumb f1) or the wrist downward (the palm Hp side of the hand H), the hemostatic device 400 maintains a state in which the auxiliary member 180 is deformed by following movement of the finger or the wrist and the auxiliary member 180 applies the compressive force to the puncture site t1. Further, in the hemostatic device 400, when the patient bends a finger (for example, the thumb f1) or the wrist upward (to the side of the dorsal side Hb of the hand H), the auxiliary member 180 is deformed following movement of the finger or the wrist, and the compressive force applied from the auxiliary member 180 to the puncture site t1 is weakened. Therefore, while hemostasis is performed by the hemostatic device 400, the hemostatic device 400 can maintain a state in which an appropriate compressive force is applied to the puncture site t1 while allowing the patient to move the finger or the wrist relatively freely.

Further, the main body 120 of the covering member 110 has the first region 121 and the second region 122 which is formed in a part different from the first region 121 and in which the inflatable member 160 is disposed. The second region 122 has the support member 125 having a higher rigidity than that of the first region 121. Therefore, the hemostatic device 400 can prevent the inflatable member 160 from rising from the dorsal side Hb of the hand H of the patient by the support member 125 while hemostasis is performed. As a result, in the hemostatic device 400, the inflatable member 160 can suitably apply a compressive force to the puncture site t1 in a state of being attached to the hand H of the patient.

Further, the first region 121 of the main body 120 is disposed to surround the periphery of the second region 122 of the main body 120. The support member 125 is inserted into the insertion portion 128a disposed on the outer surface side of the second region 122. Since the support member 125 is surrounded by the first region 121 which is more flexible than the support member 125, when the inflatable member 160 located in the second region 122 is inflated, a force expanding from the inside to the outside of the inflatable member 160 is hardly transmitted from the support member 125 to the first region 121. Therefore, the hemostatic device 400 can suppress formation of a gap between the surface of the hand H of the patient and the main body 120 at the peripheral edge of the main body 120. Further, by disposing the support member 125 to cover the outer surface side of the second region 122 of the main body 120, it is possible to effectively prevent the inflatable member 160 from rising from the dorsal side Hb of the hand H of the patient. Further, the hemostatic device 400 can be equipped with the support member 125 by a simple operation of inserting the support member 125 into the insertion portion 128a.

Further, the support member 125 has the curved portion 125a formed on the first arm portion 130 side of the support member 125. For this reason, in the support member 125, when the inflatable member 160 is inflated, a direction in which the inflatable member 160 applies a compressive force to the hand H of the patient is directed in an oblique direction toward the puncture site t1. For this reason, the hemostatic device 100 can more effectively apply a compressive force to the puncture site t1. Further, when the curved portion 125a of the support member 125 wraps the end portion of the inflatable member 160 on the first arm portion 130 side, the inflatable member 160 directs the compressive force toward the center side of the support member 125, and thus the compressive force of the second inflatable portion 180 is inhibited from escaping to the outside of the support member 125. As a result, even in a case where a gap is generated between the first arm portion 130 and the hand H of the patient, when the curved portion 125a presses the second inflatable portion 180 against the skin, it is possible to prevent the first inflatable portion 170 from rising, and it is possible to suppress a decrease in the compressive force of the inflatable member 160 applied to the puncture site t1.

Further, the inflatable member 160 is fixed to the covering member 110 on the first arm portion 130 side. Specifically, the second inflatable portion 180 of the inflatable member 160 is fixed to the covering member 110 on the first arm portion 130 side. For this reason, the inflatable member 160 (second inflatable portion 180) is fixed to the covering member 110 on the first arm portion 130 side, so that when the inflatable member 160 inflates, the direction in which the inflatable member 160 applies the compressive force to the hand H of the patient is directed in an oblique direction toward the puncture site t1. Therefore, the hemostatic device 100 can more effectively apply the compressive force to the puncture site t1.

Further, the inflatable member 160 is fixed to the covering member 110 on the curved portion 125a side of the support member 125. For this reason, in the inflatable member 160 (the first inflatable portion 170 and the second inflatable portion 180), position shift with respect to the curved portion 125a of the support member 125 of the inflatable member 160 is prevented by securing the inflatable member 160 and the support member 125, and thus a compressive force of the curved portion 125a on a position close to the first arm portion 130 becomes stronger. In this way, even when a gap is created between the surface of the first arm portion 130 and the surface of the hand H due to bending of the thumb f1 or the wrist of the patient, the hemostatic device 100 can prevent a decrease in the compressive force by the inflatable member 160.

Further, the auxiliary member 180 is a second inflatable portion 180 configured to be inflated by injection a fluid, and the lumen 181 of the second inflatable portion 180 communicates with the lumen 171 of the first inflatable portion 170. Since the auxiliary member 180 includes the second inflatable portion 180 which is inflatable, it is possible to improve the following property of the second inflatable portion 180 to movement of the hand H. Further, since the lumen 171 of the first inflatable portion 170 and the lumen 181 of the second inflatable portion 180 communicate with each other, the first inflatable portion 170 and the second inflatable portion 180 can be easily inflated.

Further, the first inflatable portion 170 and the second inflatable portion 180 have communication holes 172 and 182 communicating with the lumen 171 of the first inflatable portion 170 and the lumen 181 of the second inflatable portion 180, respectively. In addition, in the first inflatable portion 170, only a certain range around each of the communication holes 172 and 182 is fixed to the second inflatable portion 180. Specifically, in the first inflatable portion 170, only a certain range around each of the communication holes 172 and 182 located near the central portion of the second inflatable portion 180 is fixed to the second inflatable portion 180. Further, the first inflatable portion 170 is not directly connected to the covering member 110, and is indirectly connected to the covering member 110 via the second inflatable portion 180. In this way, in the second inflatable portion 180, the peripheral edge of the second inflatable portion 180 is not fixed to the first inflatable portion 170, and thus the outer shape of the second inflatable portion is freely deformed. Therefore, even when the patient bends a finger (for example, the thumb f1) or the wrist upward (to the side of the dorsal side Hb of the hand H), the hemostatic device 400 can maintain a large area in which the second inflatable portion 180 compresses the first inflatable portion 170. Therefore, the hemostatic device 400 can effectively apply a compressive force to the puncture site t1 from the inflatable member 160.

Further, the hemostatic device 400 has an injection portion 191 for injecting a fluid into the inflatable member 160. A cushioning member 192 having an inflatable space is disposed between the injection portion 191 and the inflatable member 160. In the hemostatic device 400, when the patient moves the hand H while the hemostatic device 400 is attached to the patient, the inflatable member 160 (the first inflatable portion 170 and the second inflatable portion 180) that compresses the puncture site t1 of the hand H is deformed. When there is no escape place for air in the lumen of the inflatable member 160 (the lumen 171 of the first inflatable portion 170 and the lumen 181 of the second inflatable portion 180) during deformation of the inflatable member 160, deformation of the inflatable member 160 is hindered. Therefore, the patient has a limited movable range of the hand H. The cushioning member 192 included in the hemostatic device 400 allows air to move from the lumen of the inflatable member 160 to the cushioning member 192 when the patient moves the hand H. Therefore, it is possible to prevent the movable range from being restricted by the inflatable member 160 when the patient moves the hand H. Note that when the patient returns the hand H from the deformed state to the original state, air moves from the cushioning member 192 to the inflatable member 160. Therefore, the compressive force can be effectively applied to the puncture site t1 from the inflatable member 160.

### (First modification)

Next, a description will be given of modifications of the hemostatic device according to the exemplary embodiment not falling under the scope of the claims as described above. In description of the modifications, detailed description of the configuration, etc. previously described in the exemplary embodiment will be omitted. In addition, content not particularly described in the description of the modifications can be regarded as the same as that in the first embodiment.

Fig. 12 illustrates a hemostatic device 500 according to a first modification. The hemostatic device 500 according to the first modification has a securing auxiliary portion 529 disposed in the main body 120 of the covering member 110. The securing auxiliary portion 529 is disposed at a position different from a position where the first arm portion 130 is disposed and a position where the second arm portion 140 is disposed in the main body 120. Specifically, the securing auxiliary portion 529 is disposed at a position facing the second arm portion 140 with the main body 120 interposed therebetween.

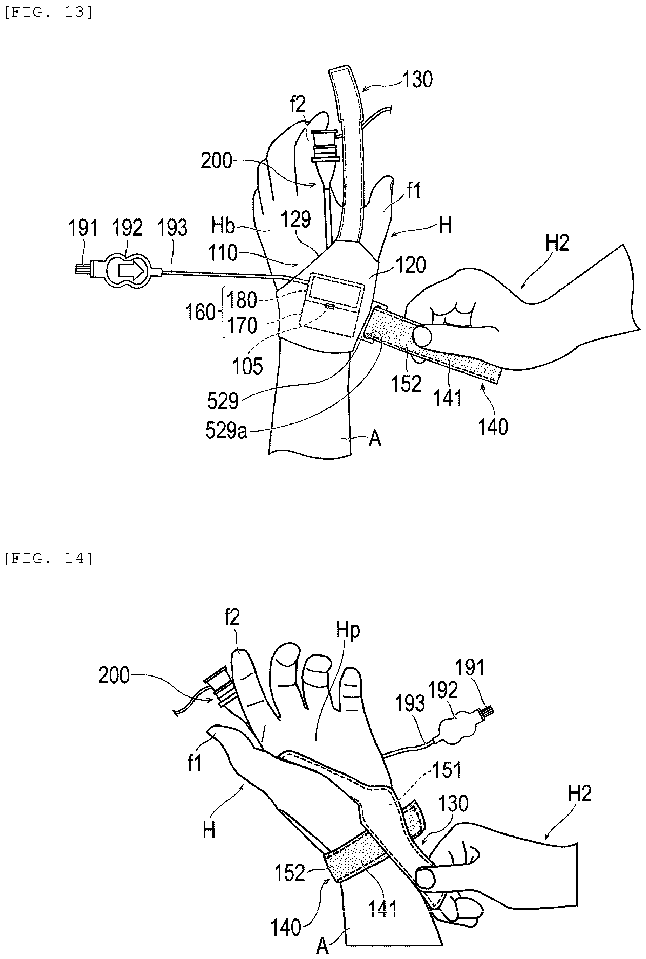

The securing auxiliary portion 529 is made of a material harder than that of the main body 120. A hole 529a through which the belt portion 141 of the second arm portion 140 can be inserted is formed in the securing auxiliary portion 529. As illustrated in Fig. 12, the hole 529a is tilted at an angle along a side portion of the main body 120 (tilted from a diagonally upper left side to a diagonally lower right side in the figure) in a state of not being attached to the hand H of the patient. Since the hole 529a is tilted in this way, as illustrated in Fig. 13, the operator, etc. can easily insert the belt portion 141 of the second arm portion 140 into the hole 529a at the time of attaching the hemostatic device 500 to the patient.

The securing auxiliary portion 529 may have a hook structure that includes a hole and a slit communicating with the hole instead of the hole 529a. Further, the securing auxiliary portion 529 may have a hole formed in a flexible material.

For example, the hole 529a can be disposed so that one end portion of the hole 529a is located on the proximal side (right side in Fig. 12) of the curved portion 125a of the support member 125. Further, the hole 529a can be disposed so as to be substantially parallel to the first inflatable portion 170. By disposing the hole 529a in this way, a tightening force is easily transmitted to the first inflatable portion 170 when the belt portion 141 of the second arm portion 140 is wrapped around the limb of the patient.

For example, the securing auxiliary portion 529 can be connected to the main body 120 by adhesion or welding.

As illustrated in Fig. 13, at the time of attaching the hemostatic device 500 to the hand H of the patient, the operator, etc. inserts the belt portion 141 of the second arm portion 140 into the hole 529a of the securing auxiliary portion 529. The operator, etc. folds back the belt portion 141 through which the hole 529a of the securing auxiliary portion 529 is inserted toward the palm Hp side of the hand H of the patient. In a state where a part of the belt portion 141 is folded back, the operator, etc. brings the third securing portion (female side of the surface fastener) 153 disposed on the inner surface of the belt portion 141 into contact with the second securing portion (male side of the surface fastener) 152 disposed on the outer surface of the belt portion 141, thereby securing the respective securing members 152 and 153. The operator, etc. holds the belt portion 141 in a state of being wrapped around the limb of the patient by securing the respective securing members 152 and 153. As illustrated in Fig. 14, the operator, etc. can attach the hemostatic device 500 to the hand H of the patient by securing the first arm portion 130 passing between the respective fingers f1 and f2 to the second arm portion 140.

### (Second modification)

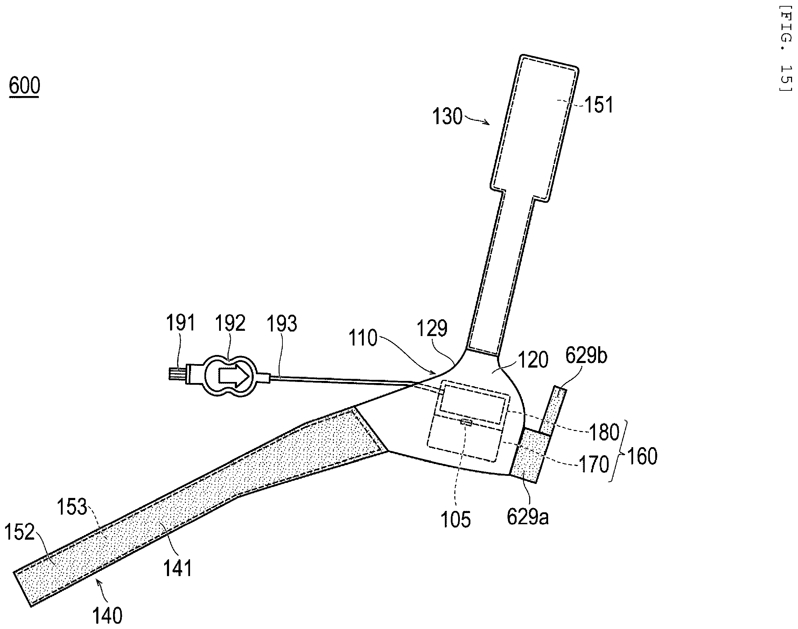

Fig. 15 illustrates a hemostatic device 600 according to the exemplary embodiment not falling under the scope of the claims as described above. The hemostatic device 600 according to the exemplary modification is different from the hemostatic device 500 according to the first modification described above in a configuration of securing auxiliary portions 629a and 629b.

The first securing auxiliary portion 629a is connected to the main body 120. A male side of a surface fastener is disposed on an outer surface (outer surface on a plan view illustrated in Fig. 15) of the first securing auxiliary portion 629a.

The second securing auxiliary portion 629b is connected to the first securing auxiliary portion 629a. A female side of the surface fastener is disposed on an outer surface (outer surface on the plan view illustrated in Fig. 15) of the second securing auxiliary portion 629b.

The respective securing auxiliary portions 629a and 629b can be connected to the main body 120 by, for example, adhesion or welding. Note that installation of the second securing auxiliary portion 629b may be omitted as appropriate.

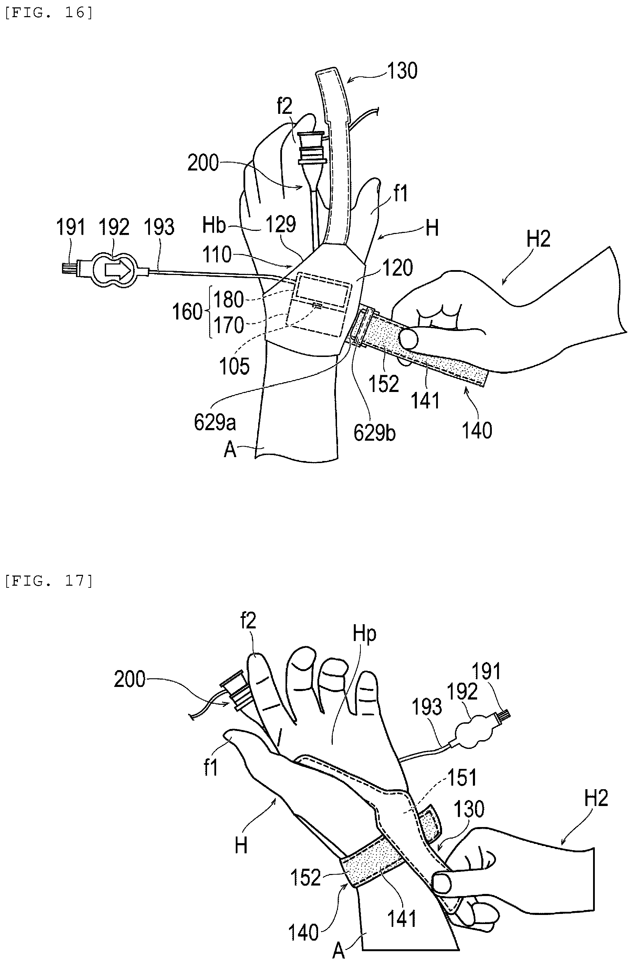

As illustrated in Fig. 16, at the time of attaching the hemostatic device 600 to the hand H of the patient, the operator, etc. secures the third securing member (female side of the surface fastener) 153 disposed on the inner surface of the belt portion 141 of the second arm portion 140 to the first securing auxiliary portion 629a. Subsequently, the operator, etc. folds back the belt portion 141 toward the palm Hp side of the hand H of the patient using a portion of the belt portion 141 secured to the first securing auxiliary portion 629a as a base point.

The operator, etc. disposes the female side of the surface fastener disposed on the outer surface of the second securing auxiliary portion 629b to cover a part of the belt portion 141 secured to the first securing auxiliary portion 629a, and secures the second securing auxiliary portion 629b to the belt portion 141.

Subsequently, the operator, etc. wraps a folded part of the belt portion 141 around the limb toward the palm Hp side. In this instance, the operator, etc. brings the third securing portion (female side of the surface fastener) 153 disposed on the inner surface of the belt portion 141 into contact with the second securing portion (male side of the surface fastener) 152 disposed on the outer surface of the belt portion 141, thereby securing the respective securing members 152 and 153. The operator, etc. holds the belt portion 141 in a state of being wrapped around the limb of the patient by securing the respective securing members 152 and 153.

As illustrated in Fig. 17, the operator, etc. can attach the hemostatic device 600 to the hand H of the patient by securing the first arm portion 130 passing between the respective fingers f1 and f2 to the second arm portion 140.

Even though the hemostatic device according to the invention has been described above through the embodiments, the invention is not limited to content described in this specification, and can be appropriately modified based on description of the scope of claims.

In the description of the embodiments, the hemostatic device for performing hemostasis at the puncture site formed on the dorsal side of the left hand has been exemplified. However, the hemostatic device can be used to perform hemostasis on a puncture site formed on a dorsal side of a right hand, a puncture site formed on a palm of the right hand, a puncture site formed on a palm of the left hand, etc.

Further, the auxiliary member is not limited to the inflatable member described in each embodiment. For example, the auxiliary member may include a member made of a resin material such as plastic, gel, etc., a member containing gel whose moisture content decreases over time to gradually reduce a compressive force, an elastic material such as a sponge-like substance, an aggregate of fibers such as cotton, metal, a member having a predetermined three-dimensional shape (sphere, ellipsoid, triangular pyramid, etc.), an appropriate combination thereof, etc.

### Reference Signs List

100, 300, 400, 500, 600 hemostatic device,
105 marker portion,
110 covering member,
120 main body,
121 first region,
122 second region,
125 support member,
125a curved portion,
128 cover member,
128a insertion portion,
130 first arm portion,
140 second arm portion,
141 belt portion,
143 support portion,
151 first securing member (securing member),
152 second securing member (securing member),
153 third securing member (securing member),
160 inflatable member,
170 first inflatable portion,
171 lumen of first inflatable portion,
180 second inflatable portion (auxiliary member),
181 lumen of second inflatable portion,
191 injection portion,
192 cushioning member,
200 introducer,
529 securing auxiliary portion,
529a hole,
629a first securing auxiliary portion (securing auxiliary portion),
629b second securing auxiliary portion (securing auxiliary portion),
c1 straight line connecting first arm portion and inflatable member,
A forearm,
B1 palmar artery,
H hand,
Hb dorsal side of hand,
Hp palm of hand,
f1 thumb (finger),
f2 index finger (finger),
t1 puncture site (site where bleeding is to be stopped).

## Claims

1. A hemostatic device (100, 300, 400, 500, 600) comprising:
a covering member (110) configured to be disposed to cover a site where bleeding is to be stopped on a hand (H) of a patient;
a securing member (151, 152, 153) configured to secure the covering member (110) in a state where the covering member (110) covers the site where bleeding is to be stopped; and
an inflatable member (160) connected to the covering member (110) and configured to be inflated by injection of a fluid,
wherein the covering member (110) includes a main body (120) to which the inflatable member (160) is connected, a first arm portion (130) protruding from the main body (120), and a second arm portion (140) protruding from the main body (120) in a direction different from a longitudinal direction of the first arm portion (130),
the first arm portion (130) is configured to be secured to the second arm portion (140) by passing between fingers of the patient in a state where the second arm portion (140) is wrapped around a limb of the patient,
the second arm portion (140) has a belt portion (141) for wrapping around the limb of the patient and a support portion (143) located on a straight line connecting the first arm portion (130) and the inflatable member (160) to connect the main body (120) and the belt portion (141), and
the belt portion (141) is configured to be secured to the support portion (143) in a state of being wrapped around the limb of the patient
**characterized in that**,
the inflatable member (160) has a first inflatable portion (170) and a deformable auxiliary member (180) having a smaller outer shape than an outer shape of the first inflatable portion (170), and
the auxiliary member (180) is disposed to overlap the first inflatable portion (170) on a side of the first arm portion (130) of the first inflatable portion (170).

2. The hemostatic device (100, 300, 400, 500, 600) according to claim 1,
wherein the main body (120) has a first region (121) and a second region (122) formed in a part different from the first region (121), the inflatable member (160) being disposed in the second region (122), and
the second region (122) has a support member (125) having a higher rigidity than a rigidity of the first region (121).

3. The hemostatic device (100, 300, 400, 500, 600) according to claim 2,
wherein the first region (121) is disposed to surround a periphery of the second region (122), and
the support member (125) is inserted into an insertion portion (128a) disposed on an outer surface side of the second region (122).

4. The hemostatic device (100, 300, 400, 500, 600) according to claim 2 or 3, wherein the support member (125) has a curved portion (125a) formed on a side of the first arm portion (130) of the support member (125).

5. The hemostatic device (100, 300, 400, 500, 600) according to claim 4, wherein the inflatable member (160) is fixed to the covering member (110) on a side of the curved portion (125a) of the support member (125).

6. The hemostatic device (100, 300, 400, 500, 600) according to one of claims 1 to 5,
wherein the auxiliary member is a second inflatable portion configured to be inflated by injection of a fluid, and
a lumen of the second inflatable portion communicates with a lumen of the first inflatable portion (170).

7. The hemostatic device (100, 300, 400, 500, 600) according to any one of claims 1 to 6, further comprising
an injection portion (191) for injecting a fluid into the inflatable member (160),
wherein a cushioning member (192) having an inflatable space is disposed between the injection portion (191) and the inflatable member (160).

## Patentansprüche

1. Hämostatische Vorrichtung (100, 300, 400, 500, 600), umfassend:
ein Abdeckelement (110), das so konfiguriert ist, dass es so angeordnet werden kann, dass es eine Stelle an einer Hand (H) eines Patienten abdeckt, an der die Blutung gestoppt werden soll;
ein Sicherungselement (151, 152, 153), das so konfiguriert ist, dass es das Abdeckelement (110) in einem Zustand sichert, in dem das Abdeckelement (110) die Stelle abdeckt, an der die Blutung gestoppt werden soll; und
ein aufblasbares Element (160), das mit dem Abdeckelement (110) verbunden und so konfiguriert ist, dass es durch die Injektion eines Fluids aufgeblasen werden kann,
wobei das Abdeckelement (110) einen Hauptkörper (120), mit dem das aufblasbare Element (160) verbunden ist, einen ersten Armabschnitt (130), der von dem Hauptkörper (120) hervorsteht, und einen zweiten Armabschnitt (140), der von dem Hauptkörper (120) in einer Richtung hervorsteht, die sich von der Längsrichtung des ersten Armabschnitts (130) unterscheidet, aufweist,
wobei der erste Armabschnitt (130) so konfiguriert ist, dass er an dem zweiten Armabschnitt (140) befestigt werden kann, indem er zwischen den Fingern des Patienten in einem Zustand hindurchgeführt wird, in welchem der zweite Armabschnitt (140) um eine Gliedmaße des Patienten gewickelt ist,
der zweite Armabschnitt (140) einen Gurtabschnitt (141) zum Umwickeln von der Gliedmaße des Patienten und einen Stützabschnitt (143) aufweist, der auf einer geraden Linie angeordnet ist, die den ersten Armabschnitt (130) und das aufblasbare Element (160) verbindet, um den Hauptkörper (120) und den Gurtabschnitt (141) zu verbinden, und
der Gurtabschnitt (141) so konfiguriert ist, dass er in einem Zustand, in dem er um das Glied des Patienten gewickelt ist, an dem Stützabschnitt (143) befestigt werden kann,
**dadurch gekennzeichnet ist, dass**
das aufblasbare Element (160) einen ersten aufblasbaren Abschnitt (170) und ein verformbares Hilfselement (180) aufweist, das eine kleinere äußere Form als eine äußere Form des ersten aufblasbaren Abschnitts (170) aufweist, und
das Hilfselement (180) so angeordnet ist, dass es den ersten aufblasbaren Abschnitt (170) an einer Seite des ersten Armabschnitts (130) des ersten aufblasbaren Abschnitts (170) überlappt.

2. Hämostatische Vorrichtung (100, 300, 400, 500, 600) nach Anspruch 1,
wobei der Hauptkörper (120) einen ersten Bereich (121) und einen zweiten Bereich (122) aufweist, der in einem Teil ausgebildet ist, der sich von dem ersten Bereich (121) unterscheidet, wobei das aufblasbare Element (160) in dem zweiten Bereich (122) angeordnet ist, und
der zweite Bereich (122) ein Stützelement (125) aufweist, das eine höhere Steifigkeit als eine Steifigkeit des ersten Bereichs (121) aufweist.

3. Hämostatische Vorrichtung (100, 300, 400, 500, 600) nach Anspruch 2,
wobei der erste Bereich (121) so angeordnet ist, dass er einen Umfang des zweiten Bereichs (122) umgibt, und
das Stützelement (125) in einen Einführungsabschnitt (128a) eingeführt ist, der an einer äußeren Oberflächenseite des zweiten Bereichs (122) angeordnet ist.

4. Hämostatische Vorrichtung (100, 300, 100, 500, 600) nach Anspruch 2 oder 3, wobei das Stützelement (125) einen gekrümmten Abschnitt (125a) aufweist, der an einer Seite von dem ersten Armabschnitt (130) des Stützelements (125) ausgebildet ist.

5. Hämostatische Vorrichtung (100, 300, 100, 500, 600) nach 4, wobei das aufblasbare Element (160) an einer Seite von dem gekrümmten Abschnitt (125a) des Stützelements (125) an dem Abdeckelement (110) befestigt ist.

6. Hämostatische Vorrichtung (100, 300, 400, 500, 600) nach einem der Ansprüche 1 bis 5,
wobei das Hilfselement ein zweiter aufblasbarer Abschnitt ist, der so konfiguriert ist, dass er durch Injektion eines Fluids aufgeblasen wird, und
ein Lumen des zweiten aufblasbaren Abschnitts mit einem Lumen des ersten aufblasbaren Abschnitts (170) in Verbindung steht.

7. Hämostatische Vorrichtung (100, 300, 400, 500, 600) nach einem der Ansprüche 1 bis 6, ferner umfassend
einen Injektionsabschnitt (191) zum Injizieren eines Fluids in das aufblasbare Element (160),
wobei ein Polsterungselement (192), das einen aufblasbaren Raum aufweist, zwischen dem Injektionsabschnitt (191) und dem aufblasbaren Element (160) angeordnet ist.

## Revendications

1. Appareil d'hémostase (100, 300, 400, 500, 600) comprenant :
un membre de recouvrement (110) configuré pour être disposé pour recouvrir un site où un saignement doit être arrêté sur une main (H) d'un patient ;
un membre de fixation (151, 152, 153) configuré pour fixer le membre de recouvrement (110) dans un état où le membre de recouvrement (110) recouvre le site où le saignement doit être arrêté ; et
un membre gonflable (160) connecté au membre de recouvrement (110) et configuré pour être gonflé par l'injection d'un fluide,
dans lequel le membre de recouvrement (110) inclut un corps principal (120) auquel le membre gonflable (160) est connecté, une première partie de bras (130) faisant saillie du corps principal (120) et une seconde partie de bras (140) faisant saillie du corps principal (120) dans une direction différente d'une direction longitudinale de la première partie de bras (130),
la première partie de bras (130) est configurée pour être fixée à la seconde partie de bras (140) en passant entre des doigts du patient dans un état où la seconde partie de bras (140) est enroulée autour d'un membre du patient,
la seconde partie de bras (140) présente une partie de sangle (141) pour l'enroulement autour du membre du patient et une partie de support (143) située sur une ligne droite connectant la première partie de bras (130) et le membre gonflable (160) pour connecter le corps principal (120) et la partie de sangle (141), et
la partie de sangle (141) est configurée pour être fixée sur la partie de support (143) dans un état d'enroulement autour du membre du patient,
**caractérisé en ce que**
le membre gonflable (160) présente une première partie gonflable (170) et un membre auxiliaire déformable (180) ayant une forme extérieure plus petite qu'une forme extérieure de la première partie gonflable (170), et
le membre auxiliaire (180) est disposé pour chevaucher la première partie gonflable (170) sur un côté de la première partie de bras (130) de la première partie gonflable (170).

2. Appareil d'hémostase (100, 300, 400, 500, 600) selon la revendication 1,
dans lequel le corps principal (120) présente une première région (121) et une seconde région (122) formée dans une partie différente de la première région (121), le membre gonflable (160) étant disposé dans la seconde région (122), et
la seconde région (122) présente un membre de support (125) ayant une rigidité supérieure à une rigidité de la première région (121).

3. Appareil d'hémostase (100, 300, 400, 500, 600) selon la revendication 2,
dans lequel la première région (121) est disposée pour entourer une périphérie de la seconde région (122), et
le membre de support (125) est inséré dans une partie d'insertion (128a) disposée sur un côté de surface extérieure de la seconde région (122).

4. Appareil d'hémostase (100, 300, 400, 500, 600) selon la revendication 2 ou 3, dans lequel le membre de support (125) présente une partie incurvée (125a) formée sur un côté de la première partie de bras (130) du membre de support (125).

5. Appareil d'hémostase (100, 300, 400, 500, 600) selon la revendication 4, dans lequel le membre gonflable (160) est fixé au membre de recouvrement (110) sur un côté de la partie incurvée (125a) du membre de support (125).

6. Appareil d'hémostase (100, 300, 400, 500, 600) selon l'une des revendications 1 à 5,
dans lequel le membre auxiliaire est une seconde partie gonflable configurée pour être gonflée par l'injection d'un fluide, et
une lumière de la seconde partie gonflable communique avec une lumière de la première partie gonflable (170).

7. Appareil d'hémostase (100, 300, 400, 500, 600) selon l'une quelconque des revendications 1 à 6,
comprenant en outre
une partie d'injection (191) pour injecter un fluide dans le membre gonflable (160),
dans lequel un membre de calage (192) ayant un espace gonflable est disposé entre la partie d'injection (191) et le membre gonflable (160).
